# EUROPEAN PATENT APPLICATION

(11) **EP 4 362 032 A1**
(43) Date of publication of application: **01.05.2024**
(21) Application number: 22204085.9
(22) Date of filing: 27.10.2022
(51) Int. Cl.: G16H 10/20, G16H 20/30, G16H 40/63, G16H 50/70

(54) **A SYSTEM AND METHOD FOR PROVIDING GUIDANCE IN RESPECT OF AN ORTHODONTIC TREATMENT INVOLVING USE OF AN ORTHODONTIC DEVICE**

(71) Applicant: Koninklijke Philips N.V., 5656 AG Eindhoven (NL)
(72) Inventor: SPELT, Hanne Adriana Alijda, Eindhoven (NL); JOHNSON, Mark Thomas, Eindhoven (NL); DE RUYTER, Boris Emmanuel Rachmund, Eindhoven (NL); VAN DEN DUNGEN, Wilhelmus Andreas Marinus Arnoldus Maria, Eindhoven (NL); GERHARDT, Lutz Christian, Eindhoven (NL)
(74) Representative: Philips Intellectual Property & Standards

(57) **Abstract**

The invention provides a computer implemented method for use in providing guidance in respect of an orthodontic treatment. The orthodontic treatment involves the use of an orthodontic device to move a subject's teeth. The method comprises receiving status information indicating usage of a first orthodontic device and receiving subject discomfort information indicating subject discomfort. The method further comprises determining a stimulus event corresponding to a change in use of the first orthodontic device (for example, inserting or removing the first orthodontic device). Stimulus discomfort information (subject discomfort information corresponding to the stimulus event) is determined and, using the stimulus discomfort information, an orthodontic treatment metric is generated and output.

## Description

### FIELD OF THE INVENTION

The present invention relates to a system and method for providing guidance in respect of an orthodontic treatment that is delivered using an orthodontic device. In particular, the present invention relates to a system and method for use in assessing treatment progress by combining temporal status information (information indicating whether the orthodontic device is being worn by the subject or not) and temporal subject discomfort information.

### BACKGROUND OF THE INVENTION

Orthodontic devices include devices for maintaining or changing the position of a subject's teeth. Orthodontic aligners are a set of tightly-fitting retainers worn over the subject's teeth usually for a fixed time period recommended by a dental professional. They are used to remedy overcrowding and/or spacing issues and can also be used to straighten teeth. Aligners are custom made; they are designed, based on the subject's teeth, to gradually change the position of the subject's teeth to a desired position. The aligners in the set are worn in successively, one after the other, each aligner being designed to move the teeth closer to the desired position.

Subjects are typically advised to wear the aligner for at least 22 hours of the day, and to follow a schedule setting out when it is time to move on from wearing one aligner and start wearing the next aligner. Non-compliance with the schedule can cause treatment to take longer than expected. If a subject's teeth have not moved to the desired position within the expected time period, it may be necessary to delay moving on to the next aligner. This may inconvenience the subject, since they will be forced to schedule additional appointment(s).

The timing for changing braces in treatment schedules is typically based on the population mean. It may be that for a particular patient the optimal time to change braces is earlier or later than the population mean.

The fit of an orthodontic device (and therefore treatment progress) can be assessed remotely by analysing images of the orthodontic device whilst it is being worn. However, subjects may find this inconvenient. Further, dental professionals may find this method of assessing treatment progress inconvenient because it is not a continuous measurement and is prone to human error.

It would be desirable to accelerate treatment success whilst minimizing inconvenience to the subject.

### SUMMARY OF THE INVENTION

The invention is defined by the claims.

According to examples in accordance with an aspect of the invention, there is provided a computer implemented method for use in providing guidance in respect of an orthodontic treatment, wherein the orthodontic treatment involves use of an orthodontic device to move a subject's teeth, the method comprising:
receiving status information indicating usage of a first orthodontic device; receiving subject discomfort information indicating subject discomfort;
determining, based on the status information, a stimulus event corresponding to a change in use of the first orthodontic device; determining stimulus discomfort information, wherein the stimulus discomfort information comprises subject discomfort information corresponding to the stimulus event; and
generating an orthodontic treatment metric based on the stimulus subject discomfort information.

The inventors have realized that measuring subject discomfort during a stimulus event - i.e., during insertion or removal of an orthodontic device - can provide a valuable insight into the fit of the orthodontic device and/or treatment progress of an orthodontic treatment involving the use of the orthodontic device. Accordingly, temporal status information is obtained to identify a time/time period corresponding to orthodontic device insertion/removal. Subject discomfort information corresponding to time/time period of the stimulus event is identified and used to generate an orthodontic treatment metric.

The status information may be temporal information indicating whether the orthodontic device is in use (i.e., whether the orthodontic device is being worn by the subject) or not. The subject discomfort information may be temporal information indicating the level of discomfort (pain) experienced by the subject.

The orthodontic device is a device, comprising a removable mouthpiece, for changing the position of the subject's teeth. The orthodontic device may be, for example, an aligner or a removable brace.

The stimulus event may relate to the insertion or removal of the first orthodontic device, or the insertion of another orthodontic device (e.g. a succeeding aligner).

Generating the orthodontic treatment metric may comprise:
determining, from the stimulus discomfort information, a level of subject discomfort associated with the stimulus event; and
comparing the level of subject discomfort associated with the stimulus event to a reference stimulus discomfort level.

The reference stimulus discomfort level may be a level of subject discomfort associated with a prior stimulus event. For example, the reference subject discomfort level may be obtained by measuring a baseline subject discomfort level when the orthodontic device is first inserted/removed. Alternatively, the reference stimulus discomfort level may be the level of subject discomfort measured when the orthodontic device was last inserted/removed. In some embodiments, the reference stimulus discomfort may be a pre-defined threshold. In general, the reference stimulus discomfort level may correspond to a reference discomfort level when the subject is in a rest state (i.e., when the orthodontic device is physiologically at rest).

The reference stimulus discomfort level may indicate an expected range for the level of discomfort associated with treatment, and determining the orthodontic treatment metric may comprise:
comparing the level of subject discomfort associated with the stimulus event to the reference stimulus discomfort level; and
in response to determining that the level of subject discomfort associated with the stimulus event is outside of the expected range, generating an orthodontic treatment metric indicating that the level of discomfort associated with the stimulus event is unexpected.

The reference stimulus discomfort level may be a fit threshold indicating a level of subject discomfort associated with an appropriate fit, and determining the orthodontic treatment metric may comprise:
comparing the level of subject discomfort associated with the stimulus event to the fit threshold; and
in response to determining that the level of subject discomfort associated with the stimulus event is equal to or greater than the fit threshold value, generating an orthodontic treatment metric indicating that orthodontic device fits the subject; and
in response to determining that the level of subject discomfort associated with the stimulus event is less than the fit threshold value, generating an orthodontic treatment metric indicating that orthodontic device does not fit the subject.

When the level of subject discomfort associated with inserting/removing/replacing the orthodontic device is higher than the fit threshold value, it may be determined that the orthodontic device is (still) exerting sufficient pressure on the subject's teeth to deliver effective treatment. When the level of subject discomfort associated with inserting/removing/replacing the orthodontic device is lower than the fit threshold value, it may be determined that the orthodontic device is not exerting sufficient pressure on the subject's teeth to deliver effective treatment. The fit threshold may vary over time.

The reference stimulus discomfort level may be an abnormality threshold. The method may comprise comparing the level of subject discomfort associated with the stimulus event to an abnormality threshold, wherein the abnormality threshold is higher than the fit threshold, the abnormality threshold indicating a maximum level of discomfort associated with normal treatment; and
in response to determining that the level of subject discomfort associated with the stimulus event is above or equal to the abnormality threshold, generating an orthodontic treatment metric indicating abnormal treatment, and
in response to determining that the level of subject discomfort associated with the stimulus event is below the abnormality threshold, generating an orthodontic treatment metric indicating normal treatment.

The reference stimulus discomfort level may be a minimum stimulus threshold. The method may comprise comparing the level of subject discomfort associated with the stimulus event to a minimum stimulus threshold; and
in response to determining that the level of subject discomfort associated with the stimulus event is below the minimum stimulus threshold, generating an orthodontic treatment metric indicating that the orthodontic device is ineffective.

The computer implemented method may further comprise:
identifying, based on the status information, a plurality of stimulus events;
determining, for each stimulus event, a level of subject discomfort associated with the stimulus event;
determining a treatment plot based on the levels of subject discomfort associated with stimulus events; and
comparing the orthodontic treatment plot and an expected treatment plot; and
generating and outputting an orthodontic treatment progress metric, wherein the treatment progress metric represents a difference between the treatment plot and the expected treatment plot.

The method may comprise generating an orthodontic treatment function representing subject discomfort over time. The method may further comprise comparing the orthodontic treatment function to an expected treatment function, wherein the expected treatment function indicates expected subject discomfort over time, according to the subject's treatment plan.

The computer implemented method may further comprise:
analysing the subject discomfort information to identify a pattern in discomfort relating to the insertion and/or removal of the orthodontic device; and
generating the status information based on the pattern in discomfort, wherein the status information comprises a predicted time of insertion and/or removal of the orthodontic device.

The computer implemented method may comprise:
receiving oral health care routine data indicating a start time and end time for using an oral health care device and
predicting status information indicating usage of the first orthodontic device based on the oral health care routine data.

The time of a stimulus event can be predicted based on historic subject discomfort information. The time of insertion and/or removal of the first orthodontic device can be predicted based on the timing of use of the oral health care device.

The computer implemented method may further comprise:
identifying a plurality of stimulus events;
determining, for each stimulus event, a level of subject discomfort associated with the respective stimulus event;
determining a rate of change of the level of subject discomfort; and
in response to determining that the rate of change of subject discomfort is lower than or equal to a progress threshold, generating and outputting a treatment progress metric indicating lack of treatment progress.

The rate of change of the level of subject discomfort can be determined, based on the levels of subject discomfort associated with multiple stimulus events. If the rate of change is higher than expected, this may indicate that treatment is progressing more quickly than expected. If the rate if change is lower than expected, this may indicate that treatment is progressing more slowly than expected. Based on this information, a treatment progress metric reflecting the relevant determination can be generated and output.

The computer implemented method may further comprise:
receiving status information indicating usage of a second orthodontic device;
identifying, based on the status information associated with the second orthodontic device, a subsequent stimulus event corresponding to a change in use of the second orthodontic device;
determining stimulus discomfort information, for the second orthodontic device, wherein the stimulus discomfort information comprises subject discomfort information corresponding to the subsequent stimulus event; and
comparing the stimulus discomfort information associated with the first orthodontic device and the stimulus discomfort information associated with the second orthodontic device.

The subsequent stimulus event is a stimulus event associated with the second orthodontic device, subsequent to the stimulus event associated with the first orthodontic device. The first and second orthodontic devices may be aligners. The first and second orthodontic devices may refer to a removable brace.

Receiving subject discomfort information may comprise receiving information indicating subject discomfort from a biological information sensing apparatus for measuring a physiological response of the subject. Sensing a subject's physiological response may facilitate an objective assessment of discomfort.

Receiving status may comprise receiving information from a sensor. The sensor may be coupled to the orthodontic device and may be configured to sense the status of the orthodontic device by measuring an environmental parameter.

The sensor may be coupled to an oral care device and may be configured to monitor use of the oral care device. The oral care device may be toothbrush.

The sensor may be coupled to a storage receptacle for storing the orthodontic device.

According to a further aspect of the invention, there is provided an orthodontic system for use providing guidance in respect of an orthodontic aligner treatment, the system comprising:
a discomfort monitor for generating subject discomfort information;
a status monitor for generating orthodontic device status information; and
a processor configured to:
   receive status information indicating usage of a first orthodontic device;
   receive subject discomfort information indicating subject discomfort;
   determine, based on the status information, a stimulus event corresponding to a change in use of the first orthodontic device;
   determine stimulus discomfort information, wherein the stimulus discomfort information comprises subject discomfort information corresponding to the stimulus event; and
   generate an orthodontic treatment metric based on the stimulus subject discomfort information.

The discomfort monitor may comprise a biological information sensing apparatus for measuring a physiological response of the subject. The biological information sensing apparatus is adapted to measure the subject's skin conductance.

The system may further comprise a second orthodontic device, and the processor may be configured to:
receive status information indicating usage of the second aligner;
determine, based on the status information, a stimulus event corresponding to a change in use of the second orthodontic device;
determine stimulus discomfort information, wherein the stimulus discomfort information comprises subject discomfort information corresponding to the stimulus event; and
compare stimulus discomfort information associated with the first aligner with stimulus discomfort information associated with the second aligner.

The processor may be configured to carry out the steps of the above-described computer-implemented method.

The orthodontic system may further comprise:
a sensor coupled to the orthodontic device and configured to sense the status of the orthodontic device by measuring an environmental parameter; or
a sensor coupled to an oral care device and is configured to monitor use of the oral care device, wherein the oral care device is preferably a toothbrush; or
a sensor coupled to a storage receptacle for storing the orthodontic device, wherein the sensor is configured to sense if the orthodontic device is inside the case; or
a display configured to display the orthodontic treatment progress metric.

According to a further aspect of the invention, there is provided a computer program product comprising instructions which, when the program is executed by a computer, cause the computer to carry out the above-described computer implemented method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram illustrating a computer implemented method according to one or more embodiments of the invention;
Fig. 2 is a diagram illustrating elements of the computer implemented method of Fig. 1;
Fig. 3 is a flow diagram illustrating an example of the computer implemented method of Fig. 1;
Fig. 4 is a flow diagram illustrating another example of the computer implemented method of Fig. 1;
Fig. 5 is a flow diagram illustrating another example of the computer implemented method of Fig. 1;
Fig. 6 is a flow diagram illustrating another example of the computer implemented method of Fig. 1; and
Fig. 7 is a schematic diagram illustrating an embodiment of an orthodontic system according to the present invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

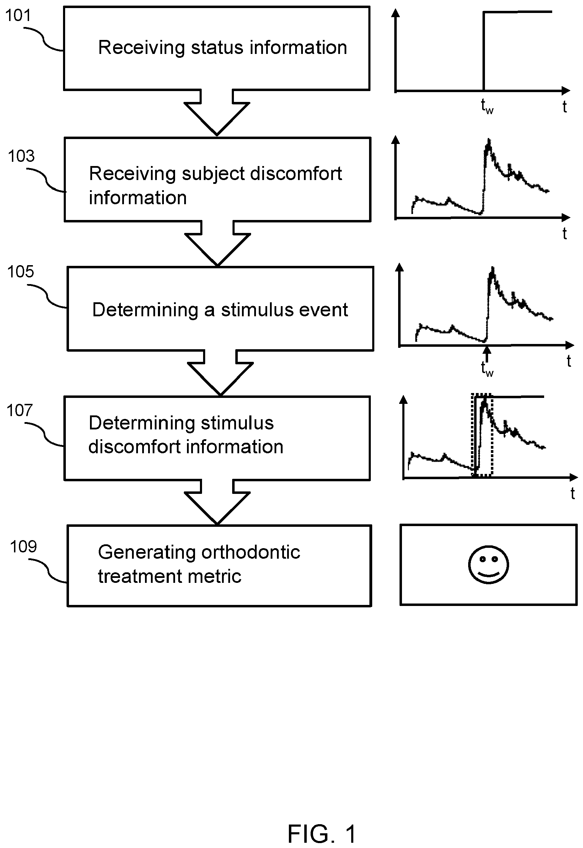

The invention provides a computer implemented method for use in providing guidance in respect of an orthodontic treatment. The orthodontic treatment involves the use of an orthodontic device to move a subject's teeth. As illustrated in Fig. 1, the method comprises receiving, 101, status information indicating usage of a first orthodontic device and receiving, 103, subject discomfort information indicating subject discomfort. The method further comprises determining, 105, a stimulus event corresponding to a change in use of the first orthodontic device (for example, inserting or removing the first orthodontic device). Stimulus discomfort information (subject discomfort information corresponding to the stimulus event) is determined 107 and, using the stimulus discomfort information, an orthodontic treatment metric is generated 109 and output (e.g. for display).

The inventors have realized that by assessing the discomfort experienced by a subject during the insertion/removal of an orthodontic device, it is possible to gain insight into how well the orthodontic device fits the subject and therefore whether the orthodontic device is capable of providing effective treatment. Further, this information can be used to assess the progress of the treatment delivered by the orthodontic device. The insertion/removal of the orthodontic device can serve as a standardized stimulus that gives context to a measured change in subject discomfort. Accordingly, in embodiments of the invention, temporal subject discomfort information is collected. Further, temporal status information - which indicates when the orthodontic device is being worn by the subject, and when it is not being worn - is collected. The subject discomfort information and status information are used to determine a level of subject discomfort associated with a stimulus event (i.e., the insertion/removal of the orthodontic device). The level of discomfort associated with a stimulus event can be used to assess the fit of the orthodontic device and/or compared to a reference level of subject discomfort (e.g. an expected level of discomfort, or a previously measured level of subject discomfort) to assess treatment progress.

Monitoring treatment in this way can lead to better health outcomes, by enabling remote assessment of wearing compliance and remote detection of progress. It can also help to reduce the number of dental visits required throughout the treatment. Further, it can help to achieve a lower cost of care by facilitating dental professionals to remotely detect when an appointment is needed. Further still, monitoring treatment in this way can enable early detection of problems with treatment and thus early intervention.

The subject discomfort information and status information comprise temporal signals, which can be synchronized. When the subject inserts the orthodontic device, it can be expected that they will experience increased discomfort and the discomfort signal (e.g., the transient peak shown in connection with step 103 of Fig. 1) will reflect that increased discomfort. If the discomfort signal indicates that the increase in discomfort is too small or not present at all, it can be assumed that it was too easy for the subject to insert the orthodontic device. That is, the orthodontic device is not/no longer exerting significant pressure on the teeth. If the orthodontic device is not/no longer exerting significant pressure on the teeth, this may indicate that the treatment schedule should be adjusted. For the example, if the orthodontic device is an aligner, it may be time to move on to the next aligner.

Similarly, when it is detected that the orthodontic device has been removed, the change in the discomfort signal provides information on how much effort the subject needs to remove the orthodontic device (e.g. tighter aligners are more effective and harder to remove). Accordingly, the discomfort signal will show a decrease in the level of discomfort which provides information on how effective the aligner is. For a more effective orthodontic device, the decrease in the discomfort signal is larger compared to a less effective orthodontic device.

The level of subject discomfort associated with the stimulus event can be assessed by comparing it to a reference stimulus discomfort level. For example, the reference stimulus discomfort level may be an expected level of subject discomfort, according to the stage of treatment. This may be assessed by comparison with a predefined threshold level of discomfort, or by comparing the level of subject discomfort to an expected treatment plot indicating the level of expected discomfort associated with a stimulus event as a function of time. The threshold level of discomfort may be determined in consultation with a dentist, using a normalized population average. Alternatively, the threshold level of discomfort could be a set standard deviation from measured discomfort with or without the aligner. In other examples, the reference stimulus level may be a level of subject discomfort experienced by the subject in connection with a previous stimulus event. In this way, it is possible to analyse how subject discomfort changes over time.

The status information and subject discomfort information may be collected continuously. Alternatively, the collection of subject discomfort information may be triggered by the insertion/removal of the orthodontic device.

As illustrated in Fig. 1, the computer implemented method comprises receiving, 101, status information. The status information is shown as a binary signal that varies as a function of time. The signal is low when the orthodontic device is not being worn, and high when the orthodontic device is being worn. The vertical line indicates that the orthodontic device was inserted at time t_{w}. Next, subject discomfort information is received 103. The subject discomfort information shown in Fig. 1 is a plot of the subject's galvanic skin response as a function of time. In the next step, the subject discomfort information is analysed to identify the point in time, t_{w}, at which the orthodontic device was inserted. Further, a portion of the subject discomfort information corresponding to the insertion of the orthodontic device (i.e. stimulus discomfort information) is identified, 107. The stimulus discomfort information is analysed to assess the level of subject discomfort, and to generate an orthodontic treatment metric. In Fig. 1, the stimulus discomfort information indicates that the level of discomfort associated with the stimulus event is sufficiently high that the orthodontic device is exerting pressure on the teeth commensurate with effective treatment, without being so high that the level of discomfort is indicative of a treatment problem/abnormality. Accordingly, the orthodontic treatment metric indicates that the fit of the orthodontic device is acceptable.

In some embodiments, treatment progress may be assessed by comparing discomfort levels relating to stimulus events for difference orthodontic devices. For example, when the orthodontic device is an aligner, the orthodontic treatment involves wearing a set of aligners, each aligner in the set moving the subject's teeth closer towards the desired position. By comparing stimulus discomfort information relating to different aligners, it is possible to assess whether the aligners are working in a similar way (and delivering effective treatment). In more detail, it is expected that each aligner in the set will cause a similar level of discomfort when it is inserted/removed. The level of discomfort depends on pressure exerted on the teeth by the aligner, which in turn depends on the geometry of the aligner. By comparing the level of discomfort associated with each aligner, it is possible to determine if one aligner is causing significantly more or less discomfort than the other aligners. If one of the aligners in the set is measured to cause a different level of discomfort compared to other aligners, this may indicate that it is not working in the same way as the other aligners and thus needs to be checked by a dental professional.

In general, the status information may be obtained using a sensor configured to sense the status of the orthodontic device. Alternatively, the status information may be generated by the subject. That is, the subject may report when a stimulus event will occur or has occurred.

In embodiments in which the status information is obtained using a sensor, the sensor may be an environmental sensor coupled to the orthodontic device. For example, the sensor may be a temperature sensor configured to sense the local temperature. Because basal body temperature in the mouth is relatively high (compared to ambient temperature outside of the subject's mouth), it is possible to assess whether the orthodontic device is being worn based on temperature. When the orthodontic device is being worn by the subject, the temperature sensor will measure an elevated temperature compared to when the orthodontic device is not in use. Alternatively, the sensor may be configured to detect the presence of saliva by measuring electric conductivity or thermal conductivity. In another example, the orthodontic device may comprise a light source (e.g. a photodiode) and the sensor may be an optical sensor configured to measure changes in light illumination and/or reflection. In a yet further example, the orthodontic device may comprise two triboelectric contact portions, and a sensor configured to detect a triboelectric signal generated by the triboelectric portions when they are brought into contact. In another example, the sensor may be a mechanical pressure sensor, arranged to detect biting forces/shear forces associated with being worn.

Alternatively, the status information may be generated using a sensor coupled to a storage receptable for holding the orthodontic device. The sensor may be arranged to detect the presence/absence of the orthodontic device in the storage receptable, thereby providing information indicative of if the orthodontic device is being worn by the subject, or not.

In some other embodiments, the status information may be determined using oral healthcare routine data. The inventors have realized that the timing of a stimulus event can be determined (estimated), or predicted, based on oral healthcare routine data. In particular, the oral healthcare routine data may comprise information indicating the time at which the subject carries out an oral care procedure (e.g. brushing/flossing/irrigating their teeth). Before the subject begins the oral care procedure, they will typically remove their orthodontic device. Once the oral care procedure has been completed, the subject will re-inset their orthodontic device. Accordingly, the time of insertion and/or removal can be determined (or predicted) based on the timing (or expected timing) of the oral care procedure. The oral healthcare routine data may be received from an oral care device (e.g., a toothbrush) or from a wearable sensor (e.g., a smartwatch configured to sense a movement pattern associated with the oral care procedure). In some embodiments, oral healthcare routine data can be obtained from a remote sensor (e.g., a camera for detecting heat/movement).

In some embodiments, the status information may be determined by predicting the time of insertion and/or removal of the first orthodontic device based on the data indicating timing over which an oral health care device is used. For example, the oral health care device may be a toothbrush communicatively coupled to a software application. The method may comprise receiving information indicating a start time of using the software application and an end time of using the software application, and predicting the time of insertion and/or removal of the first orthodontic device based on the start and end time of using the software application. For example, the time of insertion may be predicted to be a few minutes (e.g., 1-3 minutes) before the start time of using the software application. The time of removal may be predicted to be a few minutes (e.g., 1-3 minutes) after the end time of using the software application.

The subject discomfort information may be obtained using a sensor configured to sense subject discomfort by monitoring physiological responses (e.g. electrodermal activity, such as galvanic skin response).

Skin conductance is mediated through electrodermal activity, i.e. sweating. This has been shown to be linked to pain experiences (see e.g. H. Storm, "Changes in skin conductance as a tool to monitor nociceptive stimulation and pain," Curr. Opin. Anaesthesiol., vol. 21, no. 6, pp. 796-804, 2008.) Accordingly, skin conductance can be used to monitor subject discomfort.

The electrodermal system behaves uniformly across the body. However, the eccrine palmar and plantar sweat glands are especially responsive to psychological stimuli, such as pain. High skin conductance levels and peaks in the skin conductance signal, reflect emotional arousal, whereas low levels and smooth signals reflect relaxation and comfort (see e.g. S. D. Kreibig, "Autonomic nervous system activity in emotion: A review," Biol. Psychol., vol. 84, no. 3, pp. 394-421, 2010.).

In painful situations, the electrodermal system becomes activated and the sweat glands fill up. This causes a reduction in skin resistance, and skin conductance consequently increases. Shortly after, the values return to their previous (baseline) levels. This produces an identifiable peak in the skin conductance signal (which may be referred to as an electrodermal response, EDR), and the amplitude of the peak illustrates the firing strength of the sympathetic nerve, which is in turn correlated with acute pain level. These peaks are stimulus-specific, appear after a short time delay (typically around 1-2s), and fall off after the event.

Skin conductance can be measured via galvanic skin response (GSR) measurement equipment.

Skin conductance can show both psychological stress and pain (discomfort). Pain and non-pain arousal events can be detected by analyzing the skin conductance signal, and identifying relevant peaks in the signal. A relevant peak might be identified for example based on the sum of the height of the leading edge in the peak, or the area under the peak curve during a certain time interval, or based on a frequency domain analysis (e.g. based on the peak exhibiting a pre-determined spectral signature or characteristics). Further metrics of the phasic and tonic components of skin conductance can be found in: J. T. Cacioppo, L. G. Tassinary, and G. G. Berntson, The handbook of psychophysiology, 3rd ed., vol. 44. New York: Cambridge University Press, 2007; and also in W. Boucsein, Electrodermal activity, 2nd ed. New York, NY: Springer Science+Business Media, 2012.

Skin conductance is not the only biological signal which is correlated with pain and stress. For example, heart rate, PPG signal, eye movement, facial expressions, or pupil dilation may also be correlated with emotional arousal. For example, for heart rate, a data series of heart rate values as a function of time may be measured, and wherein peaks in the heart rate are used in place of peaks in skin conductance for detecting pain and/or arousal events. In addition, there is a reduction of heart rate variation with pain/non-pain arousal. Therefore, one or both of both heart rate and/or heart rate variation (HRV) may be used as the biological/physiological signal in some embodiments. For heart rate, pain causes an upward spike (an elevated peak in the heart rate signal). For HRV, pain causes a downward spike (drop) in the HRV signal.

Another example is detection of facial expressions, which correlate with arousal, e.g. grimace, clench etc. A camera could be used to acquire image data of the face, and a facial analysis algorithm used to detect variations in facial expression.

Another example is measurement of electrical activity in different groups of muscles.

Another example is tracking of pupil size or movement of eyelids, e.g. by means of a camera.

Another example is measurement of blood flow, where blood flow increases responsive to arousal.

Another example is measurement of blood pressure, where blood pressure increases responsive to arousal.

In some embodiments, the level of subject discomfort (pain) may be determined based on the presence of a galvanic skin response, the magnitude of the galvanic skin response, the half-life of the galvanic skin response (i.e. how long it takes for the galvanic skin response to decrease to half its value) or the change in the galvanic skin response with respect to a reference level.

Alternatively, the subject discomfort information may be generated by the subject. That is, the subject may report the level of discomfort experienced.

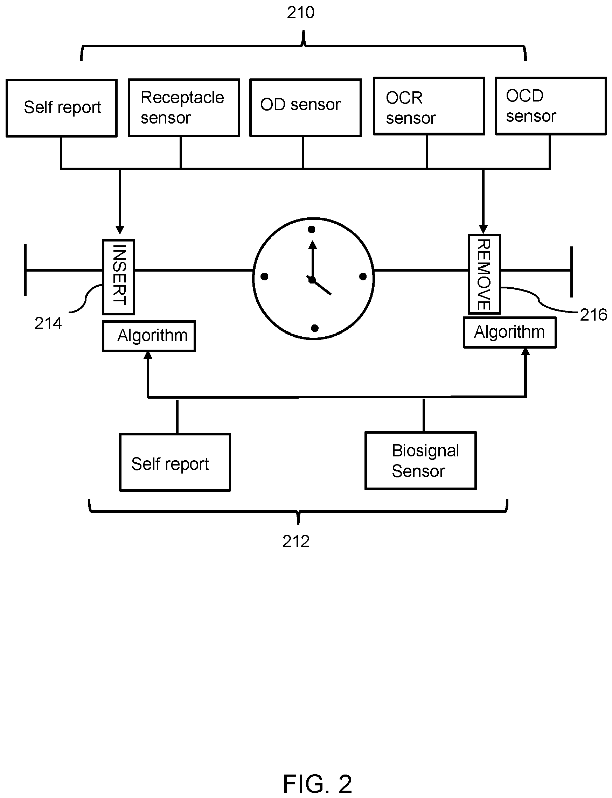

Fig. 2 is a schematic diagram illustrating the various ways in which the subject discomfort information 212 and status information 210 can be collected. As shown in Fig. 2, the status information can be self-reported or it may be obtained from a sensor (e.g. a storage receptable sensor, a sensor coupled to the orthodontic device, an oral care routine sensor, or an oral care device sensor). In some embodiments, status information 210 indicating that there has been a change in use of the orthodontic device may trigger collection of subject discomfort information 212. The data collected from the sensor/report is processed by an algorithm to generate subject discomfort information. The subject discomfort information and status information are then synchronized, to facilitate the assessment of subject discomfort information corresponding to the stimulus events 214, 216 (insertion and removal of the orthodontic device). Alternatively, the subject discomfort information 212 may be obtained without having been triggered by the detection of a change in use. In such embodiments, the subject discomfort information and status information is synchronized, so that subject discomfort information corresponding to the stimulus events 214, 216 (insertion and removal of the orthodontic device) can be identified and assessed.

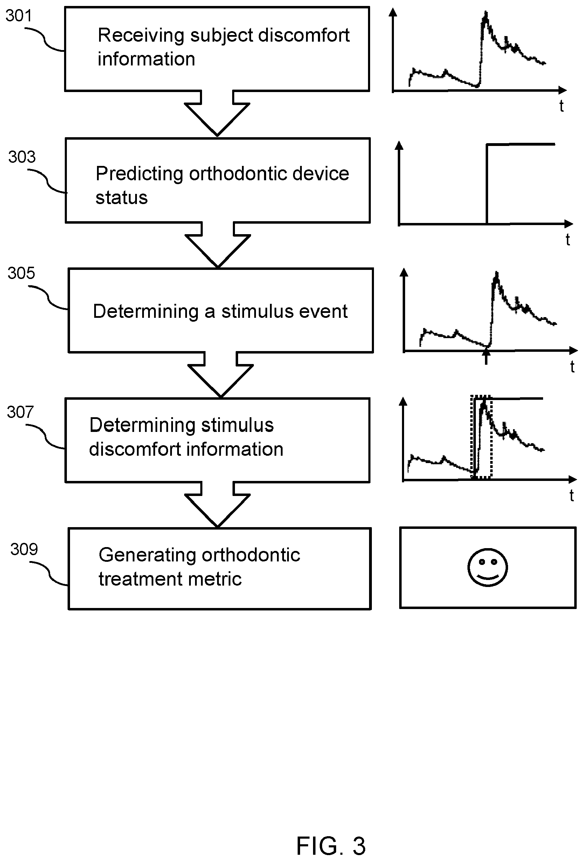

Fig. 3 illustrates an example of a computer implemented method according to the invention. In the method illustrated in Fig. 3, the orthodontic device status is predicted based on subject discomfort information. In an initial step, the computer receives, 301, temporal subject discomfort information. The inventors have realized that the subject discomfort information will typically include a recognizable pattern in the discomfort signal multiple times a day (before/after eating, before/after brushing, etc.), indicating when the subject inserts and removes the orthodontic device. Accordingly, the temporal subject discomfort information may indicate changes in a subject's level of discomfort over a relatively long time period (e.g., over a week, or multiple weeks). The subject discomfort information is processed to identify a pattern in the subject discomfort information, and to identify times at which stimulus events typically occur. In this way, the computer predicts, 303, orthodontic device status information. That is, the computer predicts the times at which the subject is wearing/has worn the orthodontic device. Based on the predicted orthodontic device status and the subject discomfort information, the computer identifies, 307, a stimulus event and stimulus discomfort information corresponding to that event (i.e., stimulus discomfort information). The method further comprises generating, 309, an orthodontic treatment metric based on the subject discomfort information.

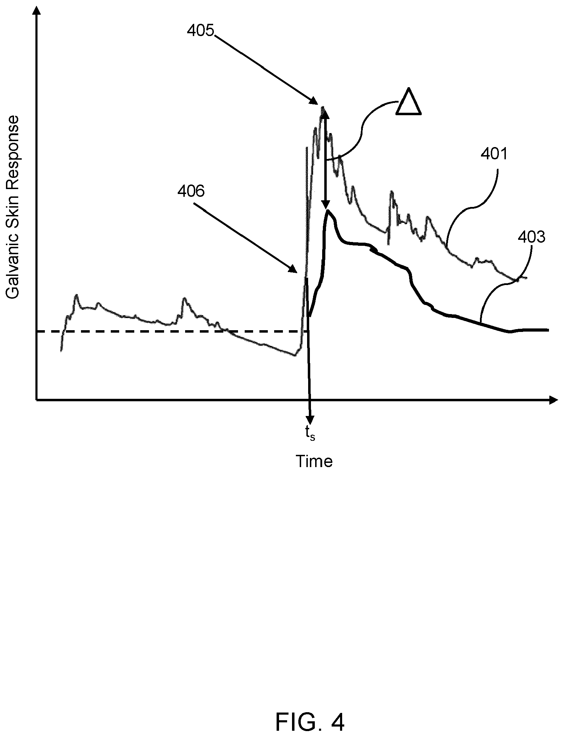

Fig. 4 shows an example of a subject discomfort information collected by a biosignal sensor. The biosignal sensor is adapted to measure skin conductance, which is indicative of subject discomfort. The first biosignal 405 comprises data collected in connection with a first orthodontic device. The dashed line shows a baseline level of subject discomfort (i.e. subject discomfort before the orthodontic device is worn). At the time tₛ, the orthodontic device is inserted into the subject's mouth. It can be seen that, in response to the stimulus event at time tₛ, the magnitude of the biosignal increases significantly. The second biosignal 407 comprises data collected in connection with the first orthodontic device at a later time period, after e.g. a few weeks have passed. The second biosignal 406 is relatively lower in magnitude. The first biosignal 405 and the second biosignal 407 can be compared to determine whether the first orthodontic device is still providing effective treatment. If the peak associated with the second biosignal 407 is significantly lower than the peak associated with the first biosignal 405, this may indicate that the first aligner is no longer providing effective treatment. For example, the magnitude of each peak and/or the difference in peak magnitudes, Δ, can be determined. The difference is magnitudes, Δ, represents a level of tooth movement brough about by the orthodontic device. Accordingly, by monitoring Δ it is possible to assess treatment progress.

The orthodontic treatment metric may be used to assist in assessing aligner fit and/or treatment progress, based on the relative peak magnitudes/change in peak magnitude. For example, if the magnitude of the peak of the second biosignal 407 is significantly lower than the peak associated with the first biosignal, a treatment metric indicating that the second aligner does not have a good fit may be generated. If the difference in peak magnitudes is lower than expected, a treatment progress metric indicating poor treatment progress may be generated.

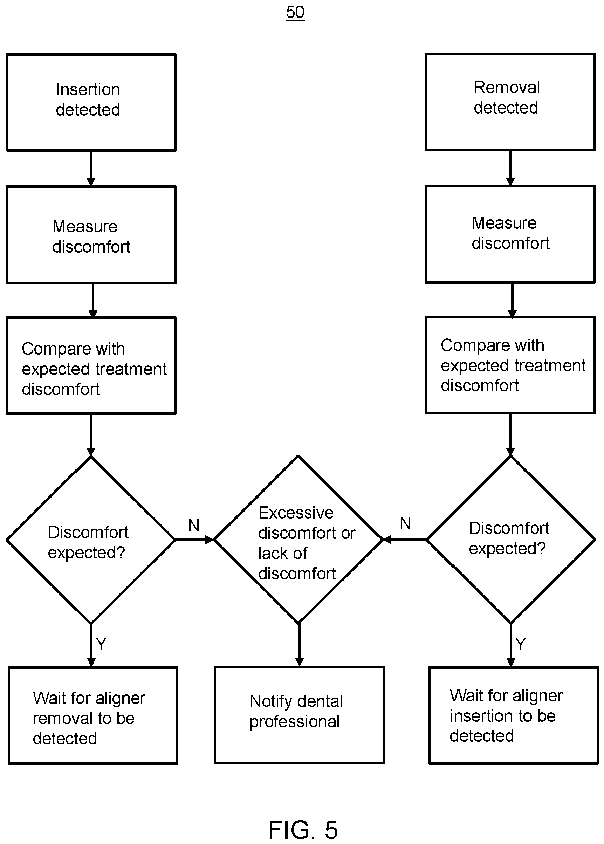

Fig. 5 is a flow diagram illustrating another example of a computer implemented method 50. In this method, the computer receives status information and determines that a stimulus event has occurred. In the left-hand column, the stimulus event is insertion of the orthodontic device. In the righthand column, the stimulus event is removal of the orthodontic device. In both cases, detecting that the stimulus event has occurred causes a measurement of subject discomfort to be carried out. In this way, the method determines stimulus discomfort information. The stimulus discomfort information indicates a level of discomfort experienced by the subject in response to the stimulus event. It is possible to assess treatment progress by comparing the level of discomfort experienced by the subject in response to the stimulus event to an expected level of discomfort, in view of the stage of treatment. For example, the expected treatment progress may be a function defining expected subject discomfort. Treatment progress can also be assessed by comparing a change in the level of subject discomfort over time to an expected change in subject discomfort over time. Determining that the measured subject discomfort is different to the expected level of subject discomfort, or the expected change in subject discomfort is different to the expected change in subject discomfort, indicates that there is an issue with treatment. Accordingly, a treatment progress metric indicating that there is a problem with treatment is generated. As shown in Fig. 5, the dental professional can be notified of the orthodontic treatment metric (e.g. by communicating a display signal for displaying the orthodontic treatment metric). Monitoring treatment in this way can facilitate convenient and early identification of treatment issues.

The inventors have realized that, to assess treatment progress, it would be useful to be able to assess whether the level of discomfort experienced by the subject is excessive. This may be assessed by comparing the level of subject discomfort corresponding to the stimulus event to an abnormality threshold. The abnormality threshold is a threshold level of subject discomfort, above (or at an above) which subject discomfort is considered excessive. Excessive subject discomfort may be associated with treatment problems (such as health issues inside the mouth) and/or non-compliance of the subject.

Typically, insertion of the orthodontic device will increase the level of discomfort experienced by the subject over for a short period of time (e.g., hours). The inventors have realized that if an elevated level of subject discomfort is extended over a long time period (e.g., multiple days), this may indicate that treatment is not progressing properly, potentially due to the orthodontic device not fitting properly. Accordingly, the method may comprise obtaining stimulus discomfort information for multiple stimulus events and determining whether the level of subject discomfort is decreasing at an expected rate, or whether it is staying substantially the same. This may be assessed by determining a rate of change of stimulus discomfort level and comparing it to a progress threshold, wherein the progress threshold indicates a minimum expected rate of change. If the rate of change of the stimulus discomfort level is below the progress threshold, an orthodontic progress metric is generated indicating that there is an issue with treatment. As shown in Fig. 5, the dental professional can be notified of the treatment progress metric (e.g. by communicating a display signal for displaying the orthodontic treatment metric). Monitoring treatment in this way can facilitate convenient and early identification of treatment issues.

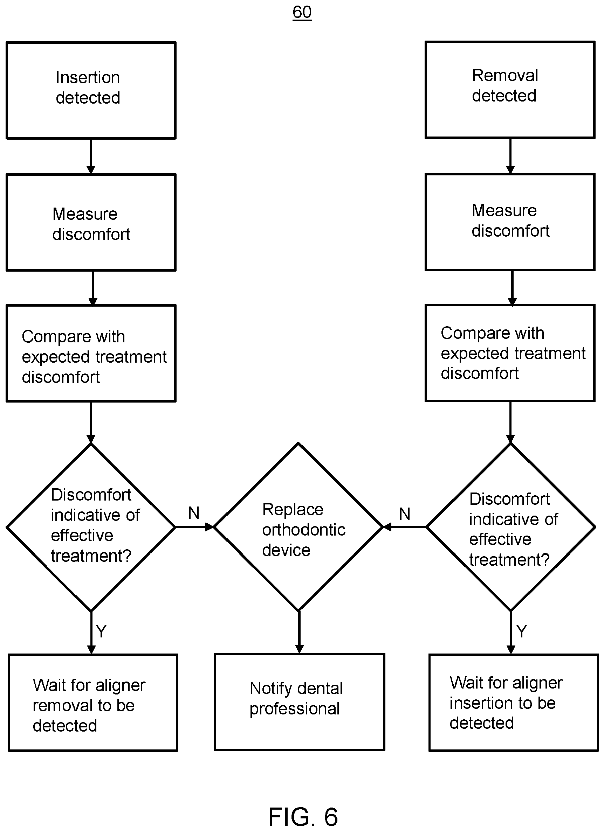

Fig. 6 is a flow diagram illustrating another example of a computer implemented method 60. In this method, the computer receives status information and determines that a stimulus event has occurred. In the left-hand column, the stimulus event is insertion of the orthodontic device. In the righthand column, the stimulus event is removal of the orthodontic device. In both cases, detecting that the stimulus event has occurred causes a measurement of subject discomfort to be carried out. In this way, the method determines stimulus discomfort information. The stimulus discomfort information indicates a level of discomfort experienced by the subject in response to the stimulus event. It is possible to assess treatment progress by comparing the level of discomfort experienced by the subject in response to the stimulus event to an expected level of discomfort, in view of the stage of treatment. For example, expected treatment progress may be a function defining expected subject discomfort. Treatment progress can also be assessed by comparing a change in the level of subject discomfort over time to an expected change in subject discomfort over time. Determining that the measured subject discomfort is different to the expected level of subject discomfort, or the expected change in subject discomfort is different to the expected change in subject discomfort, indicates that there is an issue with treatment.

The inventors have realized that, to assess treatment progress, it would be useful to assess whether the orthodontic device fits tightly enough to deliver effective treatment. This can be determined by assessing if the level of discomfort experienced by the subject is of a level indicative of effective treatment. In particular, the level of discomfort can be compared to a minimum stimulus threshold indicative of the minimum amount of subject discomfort that is expected providing the orthodontic device fits well enough to deliver effective treatment. If the level of subject discomfort corresponding to the stimulus event is lower than the minimum stimulus threshold, it may be determined that the orthodontic device does not fit well enough (e.g., no longer fits well enough) to deliver effective treatment. Accordingly, a treatment progress metric indicating that the orthodontic device should be replaced is generated. As shown in Fig. 6 the dental professional can be notified of the orthodontic treatment metric (e.g., by communicating a display signal for displaying the orthodontic treatment metric).

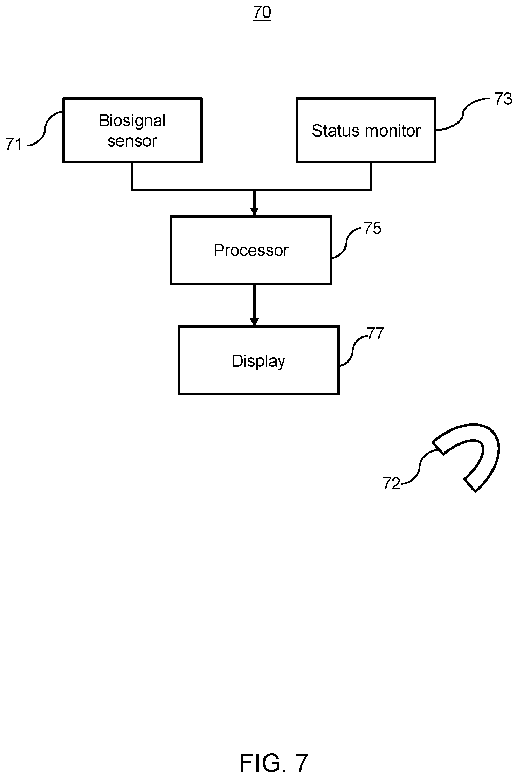

Fig. 7 is a schematic diagram illustrating the function of a system according to one or more embodiments of the invention. The system 70 comprises a first orthodontic device 72 for moving a subject's teeth, a biological information sensing apparatus comprising a biosignal sensor 71 for sensing subject discomfort and a status monitor 73 for monitoring the status of the orthodontic device 72. The biosignal sensor is configured to measure a physiological response of the subject. In particular, the biosignal sensor is configured to measure the subject's skin conductance. The system 70 further comprises a processor 75 and a display 77. The biosignal sensor 71 collects subject discomfort information, which is output to the processor 75. The processor 75 receives status information indicating usage of the first orthodontic device 72, and receives subject discomfort information indicating subject discomfort. The processor uses the information to identify a stimulus event corresponding to a change in use of the first orthodontic device. Further, the processor identifies subject discomfort information corresponding to the stimulus event, i.e. stimulus discomfort information. The processor analyses the stimulus discomfort information to generate an orthodontic treatment metric. The orthodontic treatment metric is communicated to, and displayed by, the display 77. The processor may analyse the stimulus discomfort information by comparing it to a treatment threshold and/or a fit threshold and/or an abnormality threshold.

The orthodontic device may be any removable orthodontic device comprising a mouthpiece. Preferably, the orthodontic device is an aligner or a removable brace.

The subject discomfort information may be obtained continuously, or it may be triggered by a determination that a stimulus event has occurred. Likewise, in embodiments where the timing of stimulus events is predicted (e.g. based on oral healthcare routine data, or historic subject discomfort information) measurement of subject discomfort information may be triggered to take place at the time the stimulus event is predicted to occur.

In embodiments in which subject discomfort level or a rate of change of subject discomfort level is compared to a threshold, the threshold can be varied over the course of the treatment.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer implemented method for use in providing guidance in respect of an orthodontic treatment, wherein the orthodontic treatment involves use of an orthodontic device to move a subject's teeth, the method comprising:
receiving status information indicating usage of a first orthodontic device;
receiving subject discomfort information indicating subject discomfort;
determining, based on the status information, a stimulus event corresponding to a change in use of the first orthodontic device;
determining stimulus discomfort information, wherein the stimulus discomfort information comprises subject discomfort information corresponding to the stimulus event; and
generating an orthodontic treatment metric based on the stimulus subject discomfort information.

2. The computer implemented method of claim 1, wherein generating the orthodontic treatment metric comprises:
determining, from the stimulus discomfort information, a level of subject discomfort associated with the stimulus event; and
comparing the level of subject discomfort associated with the stimulus event to a reference stimulus discomfort level.

3. The computer implemented method of claim 2 wherein the reference stimulus discomfort level indicates an expected range for the level of discomfort associated with treatment, and determining the orthodontic treatment metric comprises:
comparing the level of subject discomfort associated with the stimulus event to the reference stimulus discomfort level; and
in response to determining that the level of subject discomfort associated with the stimulus event is outside of the expected range, generating an orthodontic treatment metric indicating that the level of discomfort associated with the stimulus event is unexpected.

4. The computer implemented method of any preceding claim further comprising:
identifying, based on the status information, a plurality of stimulus events;
determining, for each stimulus event, a level of subject discomfort associated with the stimulus event;
determining a treatment plot based on the levels of subject discomfort associated with stimulus events; and
comparing the orthodontic treatment plot and an expected treatment plot; and
generating and outputting an orthodontic treatment progress metric, wherein the treatment progress metric represents a difference between the treatment plot and the expected treatment plot.

5. The computer implemented method of any preceding claim further comprising:
analysing the subject discomfort information to identify a pattern in discomfort relating to the insertion and/or removal of the orthodontic device; and
generating the status information based on the pattern in discomfort, wherein the status information comprises a predicted time of insertion and/or removal of the orthodontic device;
or
receiving oral health care routine data indicating a start time and end time for using an oral health care device and
predicting status information indicating usage of the first orthodontic device based on the oral health care routine data.

6. The computer implemented method of any preceding claim further comprising:
identifying a plurality of stimulus events;
determining, for each stimulus event, a level of subject discomfort associated with the respective stimulus event;
determining a rate of change of the level of subject discomfort; and
in response to determining that the rate of change of subject discomfort is lower than or equal to a progress threshold, generating and outputting a treatment progress metric indicating lack of treatment progress.

7. The computer implemented method of any preceding claim, further comprising:
receiving status information indicating usage of a second orthodontic device;
identifying, based on the status information associated with the second orthodontic device, a subsequent stimulus event corresponding to a change in use of the second orthodontic device;
determining stimulus discomfort information, for the second orthodontic device, wherein the stimulus discomfort information comprises subject discomfort information corresponding to the subsequent stimulus event; and
comparing the stimulus discomfort information associated with the first orthodontic device and the stimulus discomfort information associated with the second orthodontic device.

8. The computer implemented method of any preceding claim wherein receiving subject discomfort information comprises receiving information indicating subject discomfort from a biological information sensing apparatus for measuring a physiological response of the subject.

9. The computer implemented method of any preceding claim wherein receiving status comprises receiving information from a sensor, and preferably:
wherein the sensor is coupled to the orthodontic device and configured to sense the status of the orthodontic device by measuring an environmental parameter; or
wherein the sensor is coupled to an oral care device and is configured to monitor use of the oral care device, wherein the oral care device is preferably a toothbrush; or
wherein the sensor is coupled to a storage receptacle for storing the orthodontic device.

10. An orthodontic system for use providing guidance in respect of an orthodontic aligner treatment, the system comprising:
a discomfort monitor for generating subject discomfort information;
a status monitor for generating orthodontic device status information; and
a processor configured to:
receive status information indicating usage of a first orthodontic device;
receive subject discomfort information indicating subject discomfort;
determine, based on the status information, a stimulus event corresponding to a change in use of the first orthodontic device;
determine stimulus discomfort information, wherein the stimulus discomfort information comprises subject discomfort information corresponding to the stimulus event; and
generate an orthodontic treatment metric based on the stimulus subject discomfort information.

11. The orthodontic system of claim 1 wherein the discomfort monitor comprises a biological information sensing apparatus for measuring a physiological response of the subject, and preferably wherein the biological information sensing apparatus is adapted to measure the subject's skin conductance.

12. The orthodontic system of claim 10 or claim 11, further comprising a second orthodontic device, wherein the processor is configured to:
receive status information indicating usage of the second aligner;
determine, based on the status information, a stimulus event corresponding to a change in use of the second orthodontic device;
determine stimulus discomfort information, wherein the stimulus discomfort information comprises subject discomfort information corresponding to the stimulus event; and
compare stimulus discomfort information associated with the first aligner with stimulus discomfort information associated with the second aligner.

13. The orthodontic system of any preceding claim wherein the processor is configured to carry out the steps of any of claims 1-9.

14. The orthodontic system of any preceding claim further comprising:
a sensor coupled to the orthodontic device and configured to sense the status of the orthodontic device by measuring an environmental parameter; or
a sensor coupled to an oral care device and is configured to monitor use of the oral care device, wherein the oral care device is preferably a toothbrush; or
a sensor coupled to a storage receptacle for storing the orthodontic device, wherein the sensor is configured to sense if the orthodontic device is inside the case; or
a display configured to display the orthodontic treatment progress metric.

15. A computer program product comprising instructions which, when the program is executed by a computer, cause the computer to carry out the method of any of claims 1 to 9.
